# EUROPEAN PATENT APPLICATION

(11) **EP 2 441 438 A1**
(43) Date of publication of application: **18.04.2012**
(21) Application number: 11185218.2
(22) Date of filing: 14.10.2011
(51) Int. Cl.: A61K 9/00, A61K 31/00

(54) **Sachet formulations of eslicarbazepine**

(30) Priority: 15.10.2010 TR 201008460
(71) Applicant: Sanovel Ilac Sanayi ve Ticaret A.S., 34398 Istanbul (TR)
(72) Inventor: Cifter, Ümit, 34398 Istanbul (TR); Türkyilmaz, Ali, 34398 Istanbul (TR); Ergenekon, Ercan, 34398 Istanbul (TR); Öner, Levent, 34398 Istanbul (TR)
(74) Representative: Sevinç, Erkan

(57) **Abstract**

The present invention relates to a water-dispersible pharmaceutical formulation for oral administration, comprising eslicarbazepine, or a pharmaceutically acceptable salt thereof, together with at least one or more excipients.

## Description

### Field of Invention

The present invention relates to a formulation of eslicarbazepine or a pharmaceutically acceptable salt thereof. The present invention particularly relates to a sachet formulation of eslicarbazepine, which is stable and provides desired features.

### Background of Invention

Eslicarbazepine acetate is a molecule blocking voltage-gated sodium channels, providing anticonvulsant effect, and used in treating epilepsy and neuropathic pain. Its chemical designation is (S)-10-acetoxy-10,11-dihydro-5H-dibenzo[b,f]azepine-5-carboxamide, with the chemical structure illustrated below in Formula 1.

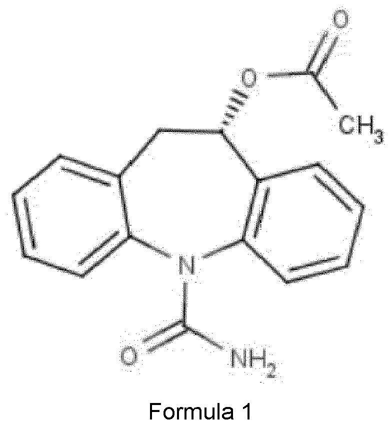

Eslicarbazepine acetate has an optically-active chiral center. It further has a solid crystalline structure, white colored, odorless, and moisture insensitive. According to Ph Eur and USP (US Pharmacopeia), eslicarbazepine acetate is dissolved in solvents at temperatures of 15 to 25°C with a pH level of an aqueous solution thereof varying in the range of 1.20 and 7.40 at 37°C ± 1°C.

Eslicarbazepine is structurally analogous to carbamazepine and oxcarbazepine, and does not contain any toxic metabolites and unwanted enantiomeric impurities.

Various formulations have been developed in relation to eslicarbazepine. There are conventional tablet formulations available, containing the eslicarbazepine acetate molecule. It is commercially-available under the tradename Exalief^{®} in 400, 600, and 800 mg tablets.

Various applications are available in the patent literature in relation to eslicarbazepine.

In the patent application WO2009054743 is disclosed a pharmaceutical formulation comprising licarbazepine acetate, a binder, and a disintegrant. According to that publication, at least part of the disintegrant is present in the granules (intragranular), and at least part of the disintegrant is extragranular.

In the patent application WO2008088233 is disclosed the use of eslicarbazepine or eslicarbazepine acetate in epilepsy, central and peripheric nervous system disorders, schizophrenia, nervous system disorders, bipolar disorders, attention disorders, anxiety disorders, neurophatic pain and related disorders, sensorimotor disorders, vestibular disorders, and nervous function alterations in degenerative and post-ischemic diseases.

In the patent application WO2006121363 is disclosed a once-a-day use of eslicarbazepine or eslicarbazepine acetate in epilepsy, central and peripheric nervous system disorders, schizophrenia, nervous system disorders, bipolar disorders, attention disorders, anxiety disorders, neurophatic pain and related disorders, sensorimotor disorders, vestibular disorders, and nervous function alterations in post-ischemic diseases.

In the patent application WO2007117166 is disclosed a process for preparing eslicarbazepine acetate by asymmetric hydrogenation of enols.

The particle size of eslicarbazepine acetate, as an active agent, directly influences its solubility and dissolution rate. Solubility and dissolution rate of a product, in turn, directly influence its bioavailability. The fact that the magnitude of the particle size of eslicarbazepine is high, keeps the bioavailability of an eslicarbazepine product to be developed from reaching to desired levels.

Another problem encountered while developing formulations of eslicarbazepine is flowability. Problems encountered with respect to the flowability of eslicarbazepine make its production difficult.

Based on the drawbacks mentioned above, a novelty is necessitated in the art of formulations of eslicarbazepine showing antiepileptic activity.

### Object and Brief Description of Invention

The present invention provides an easily-applicable sachet formulation of eslicarbazepine, eliminating all aforesaid problems and brining additional advantages to the relevant prior art.

Accordingly, the main object of the present invention is to obtain a stable formulation with antiepileptic activity.

Another object of the present invention is to provide a formulation with desired solubility and a dissolution rate, and therefore with a desired level of bioavailability, with this formulation comprising eslicarbazepine in a certain range of particle sizes.

A further object of the present invention is to develop a formulation not leading to flowability-related problems during manufacture.

Accordingly, a pharmaceutical formulation is developed which is dispersed in water for oral administration to carry out any objects, referred to above and to emerge from the following detailed description.

In a preferred embodiment according to the present invention, said novelty is carried out with eslicarbazepine, or a pharmaceutically acceptable salt thereof, together with at least one or more excipients. Eslicarbazepine, in this context, is the acetate salt of eslicarbazepine.

In a preferred embodiment according to the present invention, said excipients comprise at least one or a properly-proportioned mixture of diluents, lubricants, flavoring agents, and sweeteners.

In a preferred embodiment according to the present invention, the particle size is kept in a range of 0.1 to 500 µm.

In a preferred embodiment according to the present invention, said diluent is lactose and microcrystalline cellulose.

In another preferred embodiment according to the present invention, the proportion by weight of eslicarbazepine to lactose and microcrystalline cellulose is the range of 0.01 to 1.

In a preferred embodiment according to the present invention, lubricants are preferably magnesium stearate and/or sodium lauryl sulfate.

In a preferred embodiment according to the present invention, sweeteners comprise at least one or a properly-proportioned mixture of aspartam, sucralose, saccharine; glucose, lactose, fructose and other sugars, and mannitol, sorbitol, xylitol, erythritol, and other sugar alcohols, etc..

In a preferred embodiment according to the present invention, said flavoring agents comprise at least one or a properly-proportioned mixture of menthol, peppermint, cinnamon, chocolate, vanilin, and fruit extracts such as cherry, orange, strawberry, grape.

In a preferred embodiment according to the present invention, said pharmaceutical formulation is consisted of the following ingredients only:
a. eslicarbazepine or a pharmaceutically acceptable salt thereof at 2 to 40% by weight,
b. lactose or microcrystalline cellulose, or a properly-proportioned mixture thereof at 50 to 96% by weight,
c. sweetener at 0.1 to 5% by weight,
d. flavoring agent at 0.1 to 5% by weight,
e. lubricant at 0.1 to 5% by weight.

Another preferred embodiment according to the present invention provides a method for preparing said pharmaceutical formulation, this method comprising the steps of
a. mixing eslicarbazepine or a pharmaceutically acceptable salt thereof with lactose or microcrystalline cellulose,
b. adding sweetener and flavoring agent into the powder mixture produced above and mixing the resulting mixture,
c. adding lubricant into the mixture obtained above and mixing the resulting mixture, and
d. filling the powder mixture obtained into al - al bags in a sachet filling machine.

In a further preferred embodiment according to the present invention, said pharmaceutical formulation is in the form of sachet.

### Detailed Description of Invention

### Example

| **Ingredients** | **% amount (mg)** |
|---|---|
| eslicarbazepine acetate | 10% |
| lactose or microcrystalline cellulose | 87% |
| sodium stearate | 1% |
| sweetener | 1% |
| flavoring agent | 1% |

Eslicarbazepine acetate is mixed with lactose or microcrystalline cellulose. Into the powder mixture obtained are added a sweetener and a flavoring agent and is mixed again. To the mixture obtained is added a lubricant, and this final mixture is mixed, and is then filled into al - al bags. These bags allow to block the contact of the product with air and to achieve the desired shelf life. The particle size of eslicarbazepine acetate is in the range of 0.1 to 500 µm, thereby the solubility and dissolution rate of the formulation and therefore its bioavailability are enhanced.

Particle size of disturbution of the eslicarbazepin has measured by particle size measurement device Mastersizer Scirocco 2000A. Mastersizer Scirocco 2000A measure particle size analysis by laser diffraction.

With the invention realized, a sachet formulation of eslicarbazepine can be obtained which has surprisingly good solubility and dissolution rates, and thus high bioavailability.

The pharmaceutical compositions according to the present invention may also comprise one or more pharmaceutically acceptable excipients. Such pharmaceutically acceptable excipients include, but are not restricted to fillers, glidants, lubricants, disintegrants, surface active agents etc. and the mixtures thereof.

The present invention is used for preventing or treating epilepsy and neuropathic pain in mammalians, and particularly in humans.

It is further possible to use the following additional excipients in this formulation.

Suitable lubricants include, but are not restricted to, at least one or a mixture of sodium stearyl fumarate, polyethylene glycol, stearic acid, metal stearates, boric acid, sodium chloride benzoate and acetate, sodium or magnesium lauryl sulfate.

Suitable preservatives include, but are not restricted to, at least one or a mixture of methyl paraben and propyl paraben and salts thereof (e.g. sodium or potassium salts), sodium benzoate, citric acid, benzoic acid, butylated hydroxytoluene and butylated hydroxyanisole.

Suitable surface active agents include, but are not restricted to, at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate.

Suitable coating agents include, but are not restricted to hydroxypropyl methyl cellulose, polyethylene glycol, polyvinylpyrrolidone, polyvinylpyrrolidone-vinyl acetate copolymer (PVP-VA), polyvinyl alcohol like polymers, and all sorts of Opadry^{™}, as well as pigments, dyes, titanium dioxide and iron oxide, talk.

The present invention is hereby disclosed by referring to an exemplary embodiment hereinabove. Whilst this exemplary embodiment does not restrict the object of the present invention, the latter must be assessed under the light of the foregoing detailed description.

## Claims

**1.** A water-dispersible pharmaceutical formulation for oral administration, comprising eslicarbazepine, or a pharmaceutically acceptable salt thereof, together with at least one or more excipients.

**1.** The pharmaceutical formulation according to Claim 1, **characterized in that** said excipient comprises at least one or a properly-proportioned mixture of diluents, lubricants, flavoring agents, and sweeteners.

**2.** The pharmaceutical formulation according to any of the preceding claims, wherein said eslicarbazepine is the acetate salt of eslicarbazepine.

**3.** The pharmaceutical formulation according to any of the preceding claims, wherein the particle size of eslicarbazepine is in the range of 0.1 to 500 µm.

**4.** The pharmaceutical formulation according to any of the preceding claims, wherein said diluent is lactose and microcrystalline cellulose.

**5.** The pharmaceutical formulation according to any of the preceding claims, wherein the proportion by weight of eslicarbazepine to lactose and microcrystalline cellulose is in the range of 0.01 to 1.

**6.** The pharmaceutical formulation according to any of the preceding claims, wherein said lubricant is preferably magnesium stearate and/or sodium lauryl sulfate.

**7.** The pharmaceutical formulation according to any of the preceding claims, wherein said sweeteners comprise at least one or a properly-proportioned mixture of aspartam, sucralose, saccharine; glucose, lactose, fructose and other sugars, and mannitol, sorbitol, xylitol, erythritol, and other sugar alcohols and like.

**8.** The pharmaceutical formulation according to any of the preceding claims, wherein said flavoring agents comprise at least one or a properly-proportioned mixture of menthol, peppermint, cinnamon, chocolate, vanilin, and fruit extracts such as cherry, orange, strawberry, grape.

**9.** The pharmaceutical formulation according to any of the preceding claims, consisting of the following ingredients only:
a. eslicarbazepine or a pharmaceutically acceptable salt thereof at 2 to 40% by weight,
b. lactose or microcrystalline cellulose, or a properly-proportioned mixture thereof at 50 to 96% by weight,
c. sweetener at 0.1 to 5% by weight,
d. flavoring agent at 0.1 to 5% by weight,
e. lubricant at 0.1 to 5% by weight.

**10.** A method for preparing a pharmaceutical formulation, comprising the steps of
a. mixing eslicarbazepine or a pharmaceutically acceptable salt thereof with lactose or microcrystalline cellulose,
b. adding sweetener and flavoring agent into the powder mixture produced above in step (a) and mixing the resulting mixture,
c. adding lubricant into the mixture obtained above in step (b) and mixing the resulting mixture, and
d. filling the resulting powder mixture into bags in a sachet filling machine.

**11.** A pharmaceutical formulation according to any of the preceding claims for preventing or treating epilepsy, neuropathic pain in mammalians and particularly in humans.

**12.** The solid pharmaceutical formulation according to any of the preceding claims, wherein this formulation is in the form of sachet.
